# EUROPEAN PATENT SPECIFICATION

(11) **EP 3 801 421 B1**
(45) Date of publication and mention of the grant of the patent: **19.06.2024**
(21) Application number: 19733294.3
(22) Date of filing: 06.06.2019
(51) Int. Cl.: A61F 13/49, A61F 13/493, A61F 13/496, A61F 13/66, A61F 13/15

(54) **REVERSIBLE AND CONFIGURABLE ABSORBENT ARTICLES**
UMKEHRBARE UND KONFIGURIERBARE SAUGFÄHIGE ARTIKEL
ARTICLES ABSORBANTS RÉVERSIBLES ET CONFIGURABLES

(30) Priority: 11.06.2018 US 201862683130 P
(43) Date of publication of application: 14.04.2021
(73) Proprietor: The Procter & Gamble Company, Cincinnati, OH 45202 (US)
(72) Inventor: TALLY, Amy, Lynn, Cincinnati, Ohio 45202 (US); LUDWIG, Susan, Joy, Cincinnati, Ohio 45202 (US); ROE, Donald, Carroll, Cincinnati, Ohio 45202 (US)
(74) Representative: P&G Patent Germany
(86) International application number: PCT/US2019/035687
(87) International publication number: WO 2019/241009

(56) References cited:
- EP-A1- 0 605 012
- WO-A1-94/28841
- JP-A- S62 199 803

## Description

### FIELD

The present disclosure is generally directed to reversible and configurable absorbent articles. The absorbent articles may be used with one or more inserts.

### BACKGROUND

Absorbent articles are used to contain and absorb bodily exudates (i.e., urine, bowel movements, and menses) in infants, children, and adults. Absorbent articles may be used in hospitals for diapering infants, premature babies, and/or Neonatal Abstinence Syndrome ("NAS") babies. Premature babies, NAS babies, or other small infants may require special care by nurses and other hospital staff. These babies are oftentimes on ventilators, feeding tubes, or other life support or monitoring systems. In some instances, the babies may be positioned within incubators, also known as isolettes. The babies are typically kept in high humidity, sterile environments as they have very delicate skin that needs to be protected. It is important to the health of these premature babies that they not be handled too much and that they are maintained in a very sterile environment. Handling the babies too much may cause them stress. Some current absorbent articles for premature or NAS babies are somewhat difficult to use and are too large for very premature babies, especially in the crotch region. When the current absorbent articles are folded about their lateral axis, they typically take on a rectangular or substantially rectangular shape, thereby providing a wide crotch region with a bulky core upon loading. Additionally, some current absorbent articles for premature and NAS babies may not provide superior containment of bodily exudates and skin protection from the bodily exudates. Furthermore, some current absorbent articles do not provide full flexibility with fastener systems. Premature or NAS babies may benefit from unconventional fastening system requirements. Further, some current absorbent articles do not provide all of the features that premature or NAS baby caregivers may view as beneficial. As such, absorbent articles for premature babies, NAS babies, other babies, and other wearers should be improved.

WO 94/28841 discloses a closure system for anchoring an absorbent article on a wearer, which provides a primary line of tension around the wearer that fits predominantly within the low motion zone to anchor the absorbent core in place.

### SUMMARY

The present disclosure provides reversible and configurable absorbent articles suitable for premature and NAS babies (and other small infants, other babies, or other wearers), while not being limited to any certain sizes or uses. As discussed above, one issue with some current absorbent articles is that they are not configured for the specific needs of premature or NAS babies, in some instances, forcing nurses to handle these babies more than the nurses' desire. The configurable and reversible absorbent articles of the present disclosure overcome the disadvantages of some current premature and/or NAS baby absorbent articles by providing very narrow crotch regions, small overall dimensions, discrete and fully removable fastening members, soft skin-contacting surfaces, waist and/or side edges having curvilinear portions, gradually sloped front and back umbilical cord or surgical site notches, and optionally one or more wetness guards. In some forms, the configurable and reversible absorbent articles of the present disclosure may allow for use without any fastening members, owing to the discrete and removable fastening members. This may be desired by nurses in some instances and may reduce the need to touch or move the baby. Absorbent materials in absorbent cores of the absorbent articles may be homogeneous to allow for reversible use. Further, the absorbent cores may have an extended hourglass shape to help narrow the crotch region and provide for more fit options and leg placements.

The fully removable fastening members may allow caregivers to attach and remove the fasteners as desired at any orientation and/or at any angle. For example, the fully removable fastening members may attach to themselves (or to each other-overlapping fasteners) or may be attached to any portion of a garment-facing surface of the configurable and reversible absorbent articles and to any other portion of the garment-facing surface of the reversible configurable absorbent articles. This provides caregivers with great flexibility for various scenarios of how an absorbent article would be fastened on a premature or NAS infant, in various positions, which may be quite different than conventional taped diapers.

The absorbent articles of the present disclosure have waist and/or side edges (or outer perimeters) that comprise curvilinear portions. Babies have complex curvilinear surfaces. As such, absorbent articles with linear waist and/or side edges (or outer perimeters) may be not always be appropriate for the best fit and leakage protection, especially in the case of premature babies. Providing curvilinear portions on waist edges gradually leading to a surgical site or umbilical cord notch on the lateral midpoint, allows nurses a wider range of adjustment options on the baby. By providing waist and/or side edges (or outer perimeters) with curvilinear portions, the absorbent articles may have improved fit, improved leakage protection, and convey the impression of quality/intentionality to consumers.

The absorbent cores of the present disclosure may comprise multiple layers positioned within a core wrap or at least partially within a core wrap. The core wrap may comprise a first layer comprising a superabsorbent material (e.g., superabsorbent polymers) and optionally cellulosic fibers, a hydrophilic nonwoven material or hydrophilic material, a second layer comprising a superabsorbent material and optionally cellulosic fibers, and a masking material or a masking nonwoven material. The layers may be organized in the listed order from most proximal to the topsheet to most proximal to the backsheet. The masking material may form a portion of the core wrap most proximate to the backsheet. This multi-layer absorbent core design may be configured to deliver the absorbency and fit requirements for premature babies by enabling capacity without bulk, superabsorbent material presence for dryness, and improved urine visualization and softness optimization. The first layer may have enough superabsorbent material to enable topsheet dryness, but still may have enough cellulosic fibers to provide fast bodily exudate acquisition and a soft wearer-facing surface feel. The second layer may have more superabsorbent material than the first layer to provide a high storage capacity for bodily exudates, while also being thin and, thereby, less bulky. The hydrophilic nonwoven material or hydrophilic material between the first and second layers may provide capillary continuity between the first and second layers so that bodily exudates may easily transfer from the first layer to the second layer. The masking material may aid in the softness of the absorbent article from the garment-facing side by masking the potentially gritty feel of the superabsorbent material in the first and/or second layers. The masking material may be a nonwoven material, a film, a foam, or other suitable material. Any of the first layer, the hydrophilic material, the second layer, and the masking material may be formed of more than one layer.

The absorbent articles of the present disclosure may be used with one or more inserts. Inserts may lessen the need for a full change of an absorbent article which leads to less movement of the baby. This is highly desired in a premature or NAS baby context. Nurses may position one or more inserts on a wearer-facing surface of an absorbent article. If more than one insert is used, the inserts may form a stack of inserts on the wearer-facing surface of the absorbent article. At least some of, or all of the inserts may comprise a liquid permeable topsheet, a liquid impermeable backsheet, and an absorbent core positioned therebetween. An optional acquisition material and/or distribution material may be positioned intermediate the topsheet and the absorbent core. When the baby discharges bodily exudates, a nurse may remove the most wearer-facing insert without changing the entire absorbent article.

The present disclosure is directed, to an absorbent article as defined in claim 1 and comprising a chassis comprising a topsheet, a backsheet, and an absorbent core which may comprise a core wrap and positioned at least partially intermediate the topsheet and the backsheet. The absorbent article comprises a central lateral axis, a central longitudinal axis, a first waist edge comprising a first curvilinear portion, a second waist edge comprising a second curvilinear portion, and may comprise a first side edge comprising a third curvilinear portion, and a second side edge comprising a fourth curvilinear portion. The first side edge is positioned on a first side of the central longitudinal axis with the second side edge positioned on a second side of the central longitudinal axis. The first waist edge is positioned on a first side of the central lateral axis with the second side edge positioned on a second side of the central longitudinal axis. The core wrap may at least partially or fully encapsulate: a first layer comprising a superabsorbent material and optionally a fibrous material, a hydrophilic material or a hydrophilic nonwoven material, a second layer comprising a superabsorbent material and optionally a fibrous material, and a masking material. The materials may be in the order listed within the core wrap, with the first layer being most proximal to the topsheet, and the masking material being most proximal to the backsheet. The masking material may form a portion of the core wrap most proximate to the backsheet.

The various absorbent articles may be placed in packages. The packages may be sold in arrays or on-line arrays.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a front perspective view photograph of an absorbent article of the present disclosure in an in-use configuration;
Fig. 2 is a side view photograph of the absorbent article of Fig. 1 in the in-use configuration;
Fig. 3 not within the scope of the present invention is a plan view of an absorbent article of the present disclosure, garment-facing surface facing the viewer, and with removable fastening members attached to an outer cover nonwoven material;
Fig. 3A is a plan view of an absorbent article of the present disclosure comprising one or more lines of perforations;
Fig. 3B is a plan view of an absorbent article of the present disclosure comprising one or more lines of perforations;
Fig. 4 is a plan view of an absorbent article chassis of the present disclosure, wearer-facing surface facing the viewer, and without the removable fastening members attached thereto;
Fig. 5 is a plan view of a second surface of a removable fastening member;
Fig. 6 is a plan view of a first surface of the removable fastening member of Fig. 5;
Fig. 7 is a cross-sectional view of the removable fastening member taken about line 7-7 of Fig. 6;
Fig. 8 is a plan view of an example absorbent article chassis of the present disclosure with wetness guards and leg cuffs, wearer-facing surface facing the viewer;
Fig. 9 is a photograph of an example wetness guard of the present disclosure, wearer-facing surface facing the viewer;
Fig. 10 is an example cross-sectional schematic illustration of an absorbent core and an optional acquisition material of the absorbent articles herein, taken about a central lateral axis of a chassis;
Fig. 11 is another example cross-sectional schematic illustration of an absorbent core and an optional acquisition material of the absorbent articles disclosed herein, taken about a central lateral axis of a chassis;
Fig. 12 is a top view example of a shape of first and second layers, or other layers, of the absorbent cores disclosed herein;
Fig. 13 is an example cross-sectional schematic illustration of an absorbent core and optional acquisition material of the absorbent articles herein, taken about a central lateral axis of a chassis;
Fig. 14 is another example cross-sectional schematic illustration of an absorbent core and an optional acquisition material of the absorbent articles disclosed herein, taken about a central lateral axis of a chassis;
Fig. 15 is a left side view of an example absorbent article with an insert positioned thereon;
Fig. 16 is a plan view of an example insert positioned on a wearer-facing surface of an absorbent article;
Fig. 17 is a plan view of an example insert for use with an absorbent article;
Fig. 18 is a cross-sectional example illustration of the insert, taken about line 18-18 of Fig. 17;
Fig. 19 is another cross-sectional example illustration of the insert, taken about line 19-19 of Fig. 17;
Fig. 20 is a plan view of another example insert for use with an absorbent article;
Fig. 21 is a plan view of another example insert for use with an absorbent article;
Fig. 22 is an example side view of a leg cuff system with an inner cuff and an outer cuff;
Fig. 23 is an example cross-sectional illustration (without an outer cover nonwoven material and fastening members) of a chassis configuration taken about the central longitudinal axis of Fig. 3; and
Fig. 24 is an example cross-sectional illustration (without an outer cover nonwoven material and fastening members) of a chassis configuration taken about the central lateral axis of

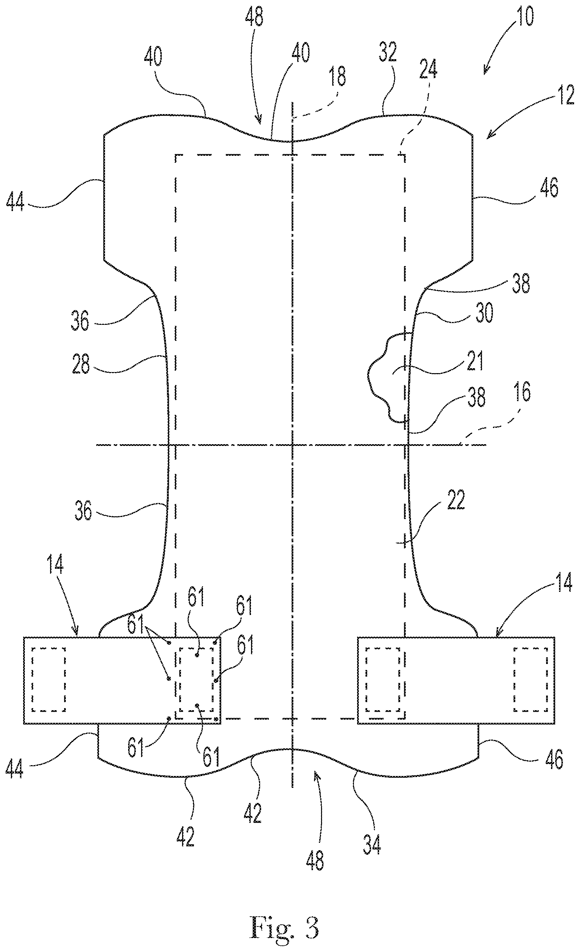

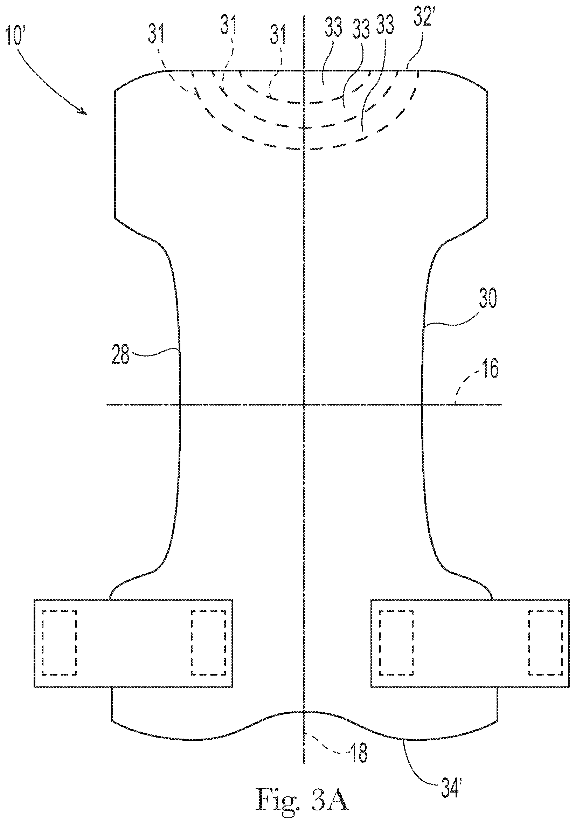

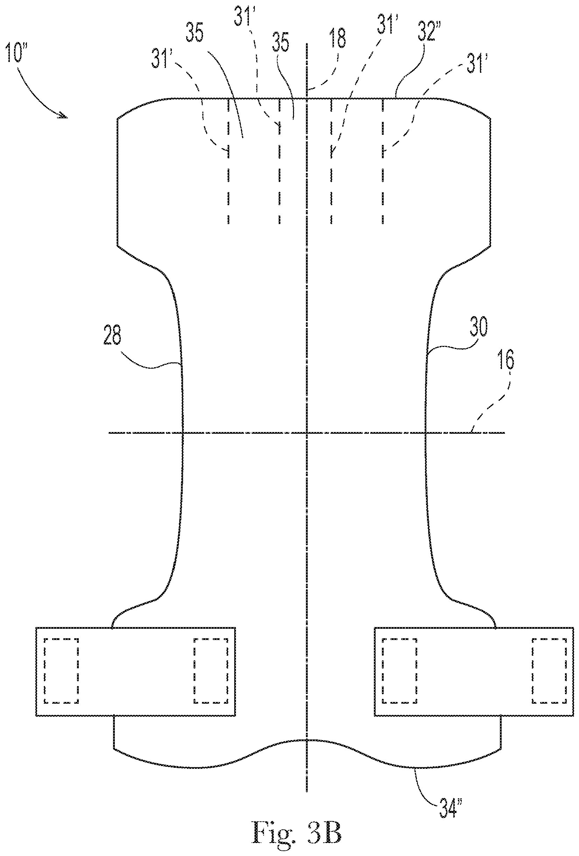

Fig. 3. The articles shown in Figs. 3a and 3b are not within the scope of the present invention.

### DETAILED DESCRIPTION

Various non-limiting forms of the present disclosure will now be described to provide an overall understanding of the principles of the structure, function, manufacture, and use of the reversible and configurable absorbent articles disclosed herein. One or more examples of these non-limiting forms are illustrated in the accompanying drawings. Those of ordinary skill in the art will understand that the reversible and configurable absorbent articles described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

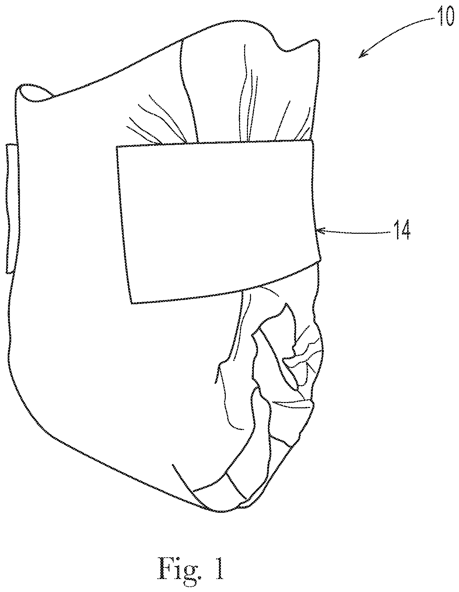

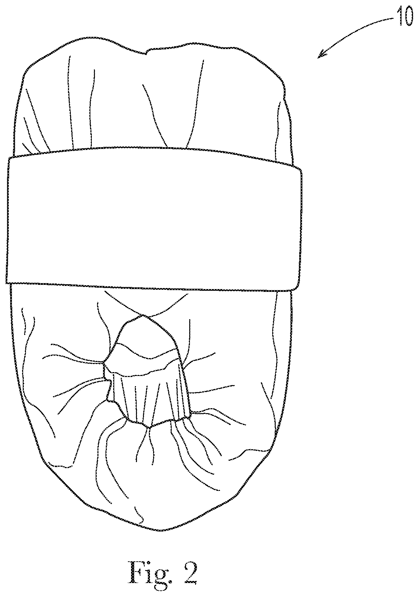

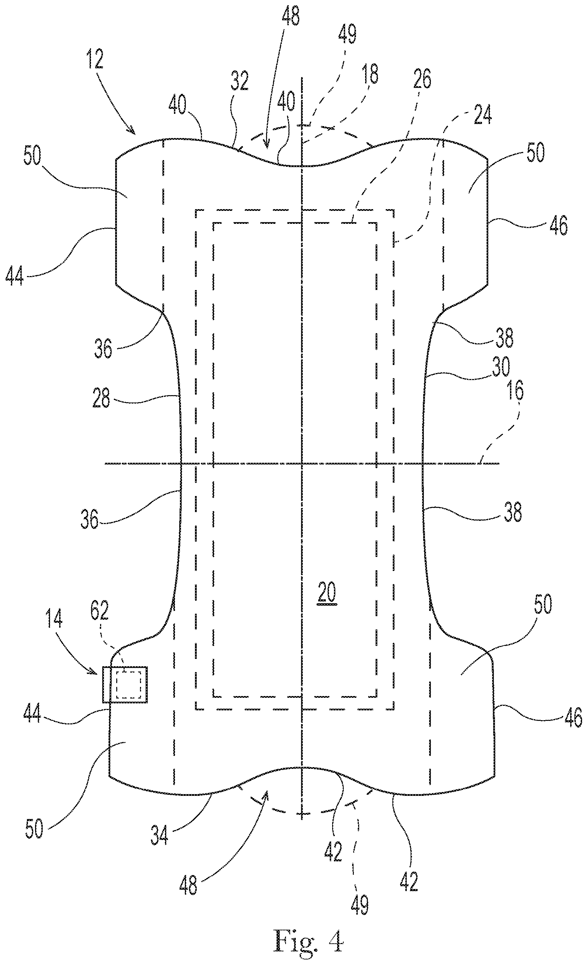

The absorbent articles of the present disclosure have waist and/or side edges each may have one or more curvilinear or arcuate portions. This enables the absorbent articles to better fit smaller infants or other wearers, due to their curvilinear surfaces. The curvilinear portions also aid in the consumer perception of softness and intentionality/customization of the absorbent articles. Fig. 1 is a front perspective view photograph of an absorbent article of the present disclosure in an in-use configuration. Fig. 2 is a side view photograph of the absorbent article of Fig. 1 in the inuse configuration. Fig. 3, not within the scope of the present invention, is a plan view of an absorbent article of the present disclosure, garment-facing surface facing the viewer, and with removable fastening members attached to an outer cover nonwoven material. Fig. 4 is a plan view of an absorbent article chassis of the present disclosure, wearer-facing surface facing the viewer, and without the removable fastening members attached thereto.

Referring to Figs. 3 and 4, an absorbent article 10 of the present disclosure comprises a chassis 12 and one or more, such as two, fully removable fastening members 14. The removable fastening members will be discussed in further detail below. The chassis 12 comprises a central lateral axis 16 and a central longitudinal axis 18. The chassis 12 comprises a liquid permeable topsheet 20 (Fig. 4) forming a portion of a wearer-facing surface of the chassis 12, a liquid impermeable backsheet 21, an optional outer cover nonwoven material 22 forming a portion of a garment-facing surface of the chassis 12, and an absorbent core 24. The absorbent core 24 may comprise a core wrap and may have a first outer surface configured to face toward a wearer-facing surface in an absorbent article and a second outer surface configured to face toward a garment-facing surface in an absorbent article. The topsheet may be apertured or non-apertured and may or may not comprise three-dimensional elements, patterns, and/or embossing. The backsheet 21 may comprise a breathable or a non-breathable film. The outer cover nonwoven material 22 may or may not comprise apertures, three-dimensional elements, patterns, and/or embossing. The chassis 12 may comprise an acquisition material 26 positioned at least partially intermediate the topsheet 20 and the absorbent core 24. The chassis 12 comprises a first side edge 28, a second side edge 30, a first waist edge 32, and a second waist edge 34. The first side edge 28 is positioned on a first side of the central longitudinal axis 18 with the second side edge 30 positioned on a second side of the central longitudinal axis. The first waist edge 32 is positioned on a first side of the central lateral axis 16 with the second waist edge 34 positioned on a second side of the central longitudinal axis. The first side edge 28 may comprise one or more first curvilinear portions 36. The second side edge 30 may comprise one or more second curvilinear portions 38. The first waist edge 32 comprises one or more first curvilinear portions 40. The second waist edge 34 comprises one or more second curvilinear portions 42. The first side edge 28 may comprise one or more linear portions 44. The second side edge 30 may comprise one or more linear portions 46. The linear portions 44 and/or 46 may be folded over material. Stated another way, the linear portions 44 and/or 46 may have two or more layers of material. The folding over of the edges of the linear portions and/or 46 results in clean, smooth edges that are visually and tactilely appealing. The first and second waist edges 32, 34 may each comprise one or more linear portions or may not comprise any linear portions.

The entire perimeter of the chassis 12 may be either cut or folded over. In some instances, the linear portions may be folded over with the remainder of the perimeter of the chassis 12 being cut. This results in clean, smooth edges that are visually and tactilely appealing. The entire perimeter of the chassis 12, whether cut or folded over, may comprise 2 or more layers. In other instances, the entire perimeter of the chassis 12 may not comprise 2 or more layers or only portions of the perimeter of the chassis 12 may comprise 2 or more layers. In this instance, the perimeter, or a portion thereof, may have 1 layer or 3 or more layers. The folded edges may be linear and the cut edges may be curvilinear or non-linear.

The chassis 12 may be substantially symmetrical about the central lateral axis 16 and/or may be substantially symmetrical about the central longitudinal axis 18 to provide reversibility. Likewise, the first side edge 28 may be substantially symmetrical to the second side edge 30 about the central longitudinal axis 18 and the first waist edge 32 is substantially symmetrical to the second waist edge 34 about the central lateral axis 16 again to provide reversibility. "Substantially symmetrical" means planned to be symmetrical, but allowing for manufacturing tolerances. In addition to the side and/or waist edges, the absorbent core 24, the optional acquisition material 26, the backsheet 21, the outer cover nonwoven material 22 (or other materials, such as a distribution layer) may be substantially symmetrical about the central lateral axis 16 and/or the central longitudinal axis 18. By having the chassis 12 and its components be substantially symmetrical about the central lateral and longitudinal axes 16, 18, the absorbent article may be fully reversible, thereby allowing a caregiver or nurse the ability to apply the absorbent article with either of the first or second waist edge on a front waist of a wearer. This reversibility is especially helpful in the context of premature infants as they may be positioned in various positions and it is desired to move them as little as possible. In some instances, the chassis 12 may not be substantially symmetrical about the central lateral axis 16 and/or the central longitudinal axis 18. In this instance, the chassis 12 may or may not still be reversible.

Referring again to Figs. 3 and 4, the first curvilinear portion 40 of the first waist edge 32 comprises one or more first concave portions and one or more first convex portions with respect to the central lateral axis 16. Likewise, the second curvilinear portion 42 of the second waist edge 34 comprises one or more first concave portions and one or more first convex portions. In some instances, the entire first and second waist edges 32, 34 may have a single concave portion and two convex portions with respect to the central lateral axis 16. The concave portions of the first and second waist edges, or portions thereof, may form umbilical cord or surgical site notches or recesses 48 in the first and second waist edges. The gradual slopes into the umbilical cord or surgical site notches or recesses 48 may be important in a premature baby context. These gradual slopes may allow nurses to easily adjust the absorbent article (i.e., pull toward the front or back) depending on the size of the infant and/or the location of a surgical site or belly button. Instead of umbilical cord or surgical site notches or recesses, the chassis 12 may have umbilical cord or surgical site projections (shown in dash) to cover the belly button or surgical site.

In some instances, the first waist edge and/or the second waist edge of an absorbent article may be provided with optional umbilical cord notches or foldable areas so that a nurse may decide whether to remove/fold over a portion of the absorbent article or not depending on the diapering situation he or she may be encountering. Referring to Fig. 3A, an absorbent article 10' may comprise one or more lines of perforations 31 proximate to a first waist edge 32' and/or a second waist edge 34'. The lines of perforations 31 may extend through the thickness of the absorbent article 10' so that a nurse may tear along one of the lines of perforations 31 to create an umbilical cord notch of varying desired size. A removable area 33 of the chassis is positioned intermediate the lines of perforations 31 and the first waist edge 32'. The removable area 33 may comprise one or more of the lines of perforations 31 depending on which line of perforations 31 the nurse chooses to tear. The lines of perforations 31 may also be provided proximate to the second waist edge 34'. The lines of perforations 31 may comprise arcuate or curvilinear portions. Referring to Fig. 3B, an absorbent article 10" may comprise two or more lines of perforations 31" proximate to the first waist edge 32" or the second waist edge 34". The lines of perforations 31" may extend through the thickness of the absorbent article 10" so that a nurse may tear along two of the lines of perforations 31" to create an umbilical cord notch of varying desired size (in both the lateral direction and the longitudinal direction). This tearing along the two lines of perforation 31" leaves behind a foldable area 35 of the chassis between the first and second lines of perforation 31". The foldable area 35 of the chassis may comprise one or more lines of perforations 31" depending on what two lines of perforations 31" the nurse tears. In such a configuration, the nurse may fold at least a portion of the foldable area 35 over the wearer-facing surface or the garment-facing surface of the absorbent article to create the umbilical cord notch. The nurse may optionally tape or otherwise attach a portion of the foldable area 35 to the wearer-facing surface or to the garment-facing surface or cut away the material with scissors, for example. The lines of perforations 31" may also be provided proximate to the second waist edge 34".

The chassis 12 may be a uni-body chassis or may have protrusions 50 (shown with dashes) attached thereto. The protrusions 50 may be attached to a main body of chassis (i.e., chassis 12 without the protrusions 50) much like ears are attached to commercially available absorbent articles, such as through the use of adhesives and/or bonding. In a non-uni-body configuration, first and second protrusions 50 may be attached proximate to the first side edge 28 and third and fourth protrusions 50 may be attached proximate to the second side edge 30. In a uni-body configuration, a first protrusion 50 may be formed in the first side edge 28, a second protrusion may be formed in the second side edge 30, a third protrusion may be formed in the first side edge, and a fourth protrusion may be formed in the second side edge 30. Each of the first, second, third, and fourth protrusions, in the uni-body configuration, are not separate elements from the chassis 12. In either configuration, at least one of, two of, three of, or all of the protrusions 50 may each have one or more additional curvilinear portions (separate from curvilinear portions in the side edges). A uni-body configuration may be desired because the absorbent article would not have seams (where components are joined to each other), thereby leading to improved softness and improved leakage protection.

The first and second side edges 28, 30 and the first and second waist edges 32, 34 may together form an outer perimeter of the chassis 12. In the context of the uni-body chassis and the non-uni-body chassis, the protrusions 50 form portions of the first and second side edges and, thereby, the outer perimeter. About 50% to about 95%, about 60% to about 95%, or about 60% to about 90%, of the outer perimeter may be curvilinear, specifically reciting all 1% increments within the specified ranges and all ranges formed therein or thereby. Only about 5% to about 50%, about 5% to about 40%, about 5% to about 30%, or about 5% to about 25%, of the outer perimeter may be linear (i.e., straight), specifically reciting all 1% increments within the specified ranges and all ranges formed therein or thereby.

On an absorbent article manufacturing line, a web having an infinite length is typically conveyed in a machine direction. These webs typically have linear side edges and are cut in a cross-machine direction to an absorbent article pitch. The one or more linear portions 44 and the one or more linear portions 46 may be portions of the linear side edges of the webs, while the curvilinear portions 36, 38, 40, 42 may be formed by additional cutting and removal of the cut portions (i.e., trim removal).

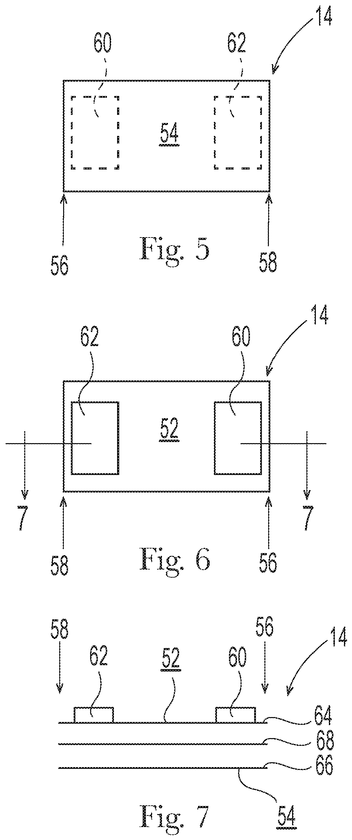

Fig. 5 is a plan view of a second surface of a removable fastening member. Fig. 6 is a plan view of a first surface of the removable fastening member of Fig. 5. Fig. 7 is a cross-sectional view of the removable fastening member taken about line 7-7 of Fig. 6. The fully removable fastening members 14 may be stretch panels that may be discrete elements from the chassis 12. Referring to Figs. 5 and 6, the fastening members 14 may each comprise a first surface 52, a second surface 54, a first end 56, and a second end 58. The first surface 52 may be opposite to the second surface 54 and the first end 56 may be opposite to the second end 58. The fastening members 14 may comprise a first fastener 60 that may be configured to engage a first portion of the outer cover nonwoven material 22 or a first portion of a landing zone (if provided) and be positioned on the first surface 52. The first fastener 60 may be configured to engage a portion of the chassis 12 on a first side of the central lateral axis 16. The chassis 12 may be free of a landing zone and any portion of the outer cover nonwoven material 22 may function as the landing zone. The fastening members 14 may comprise a second fastener 62 that may be configured to engage a second, different portion of the outer cover nonwoven material 22 and positioned on the first surface 52. The second fastener 62 may be configured to engage a portion of the chassis 12 on a second side of the central lateral axis 16. In some instances, only one fully removable fastening member 14 may be provided with a chassis 12. In this scenario, the first fastener 60 may engage a portion of the outer cover nonwoven material 22 of the chassis 12 and the second fastener 62 may engage another portion of the outer cover nonwoven material 22 or may engage a portion of the second surface 54 of the fastening member 14. The fastening members 14 may be provided in a package with one of the fasteners 60, 62 attached to portions of the outer cover nonwoven material and with the other fastener 60, 62 engaged with the topsheet 20 (or other portion of the chassis) or not engaged to any portion of the chassis 12. The other fastener 60, 62 may also be engaged with a surface 52, 54 of the fastening members to at least inhibit the fasteners 60, 62 from catching on other absorbent articles or other items. In still other instances, the fastening members 14 may be provided in a package with the chassis 12, but be separate from the chassis 12. In other instances, the fastening member 14 and the chassis 12 may be provided in separate packages.

Since the fastening members 14 are fully removable from the absorbent article 10, they can be fastened as desired by a nurse or caregiver. In some instances, the nurse or caregiver may remove the fastening members 14 from the chassis 12 and not use them if the infant is in a certain position, for example. In other instances, the nurse or caregiver may only use one of the fastening members 14 if the infant is in another certain position, for example.

Referring to Figs. 5 and 6, the first fastener 60 and the second fastener 62 may not extend to the outer perimeter of the fastening members 14. This may help prevent, or at least inhibit, rough fastener material (e.g., hooks) from contacting or irritating a wearer's skin. The fasteners 60 and 62 are illustrated as rectangular but may be any other suitable shape, such as circular or ovate, for example. In some instances, it may be desirable to have fasteners without corners to again prevent, or at least inhibit the fasteners from irritating a wearer's skin.

Referring to Figs. 3 and 4, the first fastener 60 may be attached to the garment-facing surface of the chassis 12 in an area that overlaps, or partially overlaps, the absorbent core 24, inboard, or partially inboard, of the protrusions 50. By attaching the first fastener 60 in an area that overlaps the absorbent core 24, the first fastener 60 is positioned in a low motion force zone on the chassis 12 to minimize unintended separation of, for example, hooks 64 of the first fastener 60. As a result, a nurse or caregiver may apply the absorbent article without unattaching the first fastener 60 or re-securing the first fastener 60. This can be quite desirable in a hospital setting, especially if the baby is premature. This concept may apply to both of first fasteners 60 of the fastening members 14.

In addition, the first fasteners 60 or areas of the fully removable fastening members 14 proximate to the first fasteners 60 may be additionally secured to the garment-facing surface of the chassis 12 by additional mechanical bonds, thermal bonds, ultrasonic bonds, and/or glue (together referred to as "bonds" and numbered 61 in Fig. 3), for example. Any suitable number, shape, and/or size of bonds 61 may be provided on the first fastener 60 or on areas around the first fastener. The bonds 61 may provide the first fasteners 60 or areas of the fully removable fastening members 14 proximate to the first fastener 60 with additional strength beyond merely the hooks 64 for additional resistance to disengagement. In such an instance though, the first fastener 60 may still be removable from the chassis 12. The bonds 61 may be positioned on leading and trailing edges of the hooks 64, for example, and/or on the left and right side of the hooks 64. In other instances, the bonds 61 may be positioned in corners of the first fasteners or outboard of corners of the first fasteners. The bonds 61 not only aid in securing the first fasteners 60 to the garment-facing surface of the chassis 12, but also prevent, or at least inhibit, the first fasteners 60 from partially peeling off and exposing a portion of the hooks 64, which could cause the hooks 64 to undesirably engage clothing, blankets, and/or other items. The first fastener may be positioned and attached as discussed in in this paragraph when in a package or when presented to a caregiver. The first fasteners may be fastened to the first waist region or the second waist region in this fashion. The second fasteners 62 may also comprise the bonds to prevent, or at least inhibit, unintentional opening of the second fasteners 62.

When packaged, the second fasteners 62 may engage a wearer-facing surface of the chassis 12 in the protrusions 50. Typically, the second fasteners 62 may not have the bonds 61 in that the second fasteners are intended to be opened and repositioned on the chassis 12. By initially attaching the second fasteners 62 to the wearer-facing surface of the chassis 12 in the protrusions 50, a nurse or caregiver is able to grasp an absorbent article, slide the absorbent article under an infant or premature baby without having to move the baby to unattach the second fastener 62. Stated differently, the second fastener 62 may not be underneath the infant or premature baby so that the baby does not need to be moved, rolled, and/or lifted, to remove and reattach the second fasteners 62 in an appropriate location.

Referring to Fig. 7, the fastening members 14 may comprise a first nonwoven or other substrate 64, a second nonwoven or other substrate 66, and an elastic material 68 positioned at least partially intermediate the first and second substrates 64 and 66. The elastic material 68 may comprise an elastic nonwoven material, an elastic film, and/or elastic strands, for example. The elastic material may be apertured or micro-apertured to promote breathability. In other instances, the fastening members may comprise one or more substrates and may not comprise an elastic material. In still other instances the fastening member may comprise one or more elastic nonwoven substrates.

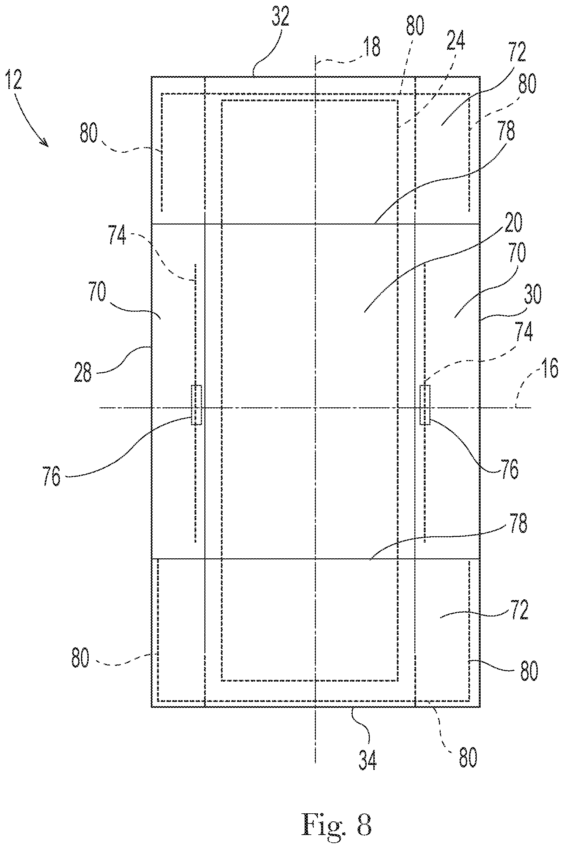

Fig. 8 is a plan view of an example absorbent article chassis 12 of the present disclosure with wetness guards and leg cuffs, wearer-facing surface facing the viewer. The fully removable fastening members 14 may be used with the absorbent article chassis 12, much like illustrated in Fig. 3 and discussed herein. The example absorbent article chassis 12 is not illustrated with the curvilinear portions on the waist and side edges 32, 34, 28, 30 for simplicity in illustration, although it will be understood that the chassis may have the same or similar shape as the chassis of Figs. 3 and 4. The absorbent article chassis 12 may comprise one or more pairs of leg cuffs 70 and one or more wetness guards 72. The leg cuffs 70 may have one or more elastic strands 74 positioned therein. The elastic stands 74 may be attached to the cuffs only in a central zone 76 proximate to the central lateral axis 16. As an example, the central zone may 76 extend between about 0.5 inches and about 2.5 inches in a smaller sized absorbent article. In the central zone 76, the elastic strand 74 may be glued or bonded to a portion of the leg cuff. In some instances, only one pair of leg cuffs may be provided. Additional features of the leg cuffs are disclosed in U.S. Patent Application Publication No. 2017/0246052, to Ludwig et al., published on August 31, 2017.

One or more wetness guards 72 may be provided on a wearer-facing surface of the chassis 12. In some instances, only one wetness guard may be provided on a longitudinally end region of the chassis 12. In other instances, two wetness guards may be provided on longitudinally opposite end regions of the chassis 12. When bodily exudates, especially urine, enter an absorbent article they may initially be absorbed in a central longitudinal region of the absorbent article or the crotch region. By design, and to inhibit the crotch region from becoming overloaded, the bodily exudates are wicked towards the longitudinal end region of the absorbent article by the acquisition material and the absorbent core. This allows the crotch region to be able to accept additional insults of bodily exudates without overloading. This also potentially makes the longitudinal end regions wet. In order to protect an infant's skin in contact with the longitudinally end regions, such as the lower back and the front waist of a wearer, wetness guards may be provided in one or both of the longitudinally end regions. The wetness guards 72 may each overlap a portion of the topsheet 20 to inhibit topsheet to skin contact and thereby reduce moisture on the skin of a wearer.

The wetness guards 72 may comprise one or more layers of material. In an instance, the wetness guards may comprise a film facing the topsheet 20 and one or more nonwoven materials facing the wearer. In other instances, the wetness guards 72 may comprise a hydrophobic nonwoven material without a film. By providing a film or a hydrophobic nonwoven material, wetness from the absorbent core or acquisition material may be shielded from contacting the lower back or the front waist of a wearer, thereby improving skin health and dryness.

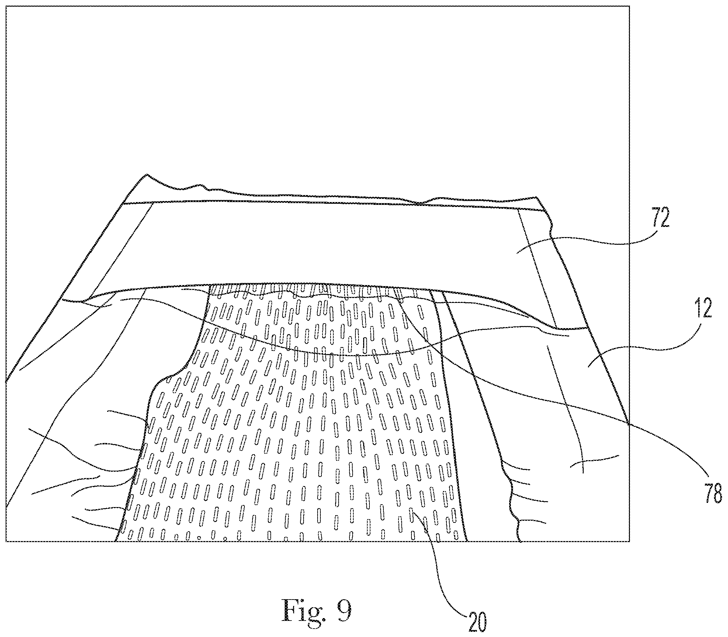

In some instances, the wetness guards 72 may be formed of portions of the backsheet film 21 and outer cover nonwoven 22 folded over the waist edges 32, 34. In other instances, the wetness guards may be discrete components attached to the chassis 12. Fig. 9 illustrates a photograph of a wetness guard 72 attached to a chassis 12, wearer-facing surface facing the viewer. The wetness guards 72 may have a free, unattached end 78 most proximate to the central lateral axis 16. The free, unattached end 78 is illustrated as linear in Fig. 9, but may comprise curvilinear portions. The wetness guards may be fully removable from the chassis 12, if desired, by providing hooks or other attachment features on the topsheet facing side of the wetness guards and/or on the topsheet. In other instances, the wetness guards 72 may be permanently bonded, adhesively attached, or otherwise attached, to the chassis 12, for example along attachment area 79. Additional features of the wetness guards are disclosed in U.S. Patent Application Publication No. 2017/0246052, to Ludwig et al., published on August 31, 2017.

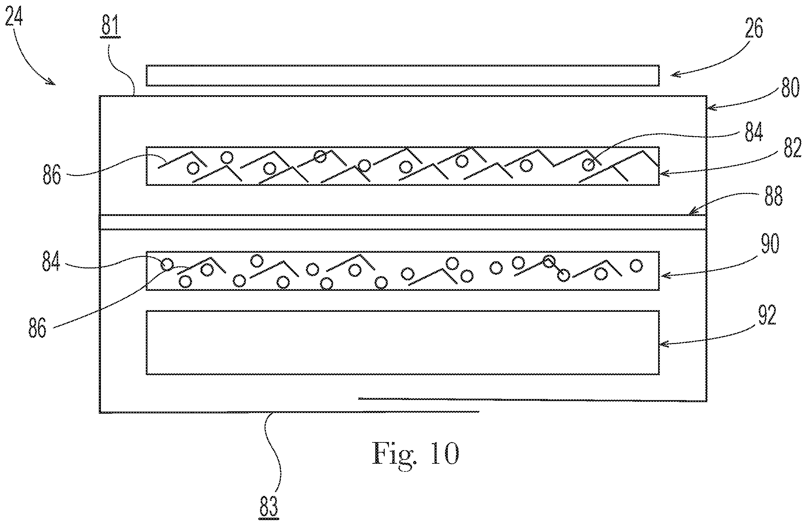

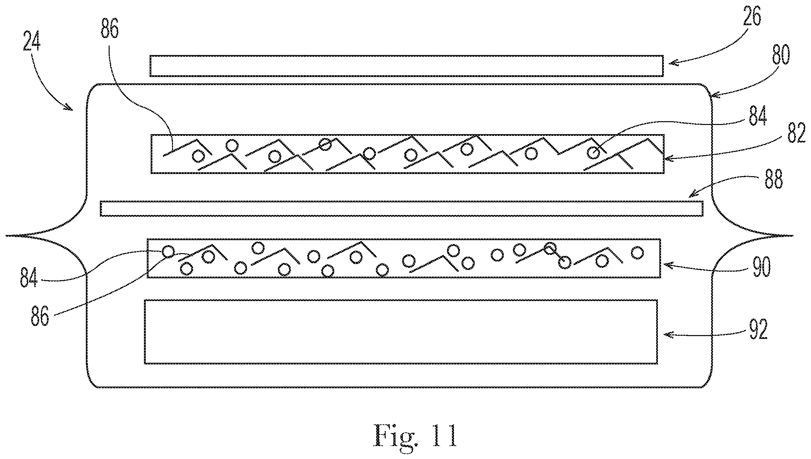

Fig. 10 is an example cross-sectional schematic illustration of an absorbent core 24 and optional acquisition material 26 of the absorbent articles herein, taken about a central lateral axis 16 of a chassis 12. Fig. 11 is another example cross-sectional schematic illustration of an absorbent core 24 and an optional acquisition material 26 of the absorbent articles disclosed herein, taken about a central lateral axis 16 of a chassis 12. The absorbent cores 24 of the present disclosure are designed to provide superior absorbency and storage with reduced bulk and improved softness from both the garment-facing side and the wearer-facing side. Further benefits of such an absorbent core are discussed above.

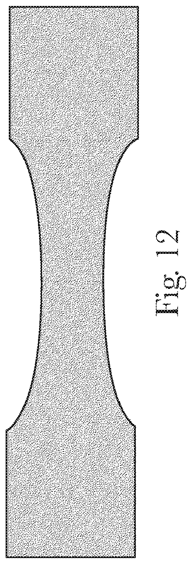

The optional acquisition material 26 may be positioned outside of a core wrap 80. The acquisition material 26 may be wider (direction along the central lateral axis 16) than the core wrap 80 or not as wide as the core wrap 80. The acquisition material 26 may be longer (direction along the central longitudinal axis 18) than the core wrap or shorter than the core wrap 80. A distribution material may or may not be provided intermediate the acquisition material 26 and the core wrap 80, or otherwise located. The acquisition material 26 and/or a distribution material may be rectangular or shaped, such as hourglass shaped, with a narrower crotch region and wider longitudinal end regions. The shape may be similar to that illustrated in Fig. 12.

The core wrap 80 may be formed of a single material (Fig. 10) or may be formed of two or more materials (Fig. 11). The core wrap 80 may also take on a C-wrap design where a first upper (closest to topsheet) material is partially wrapped around a lower material. The lower material, as discussed below, may be formed by a portion of the masking material. The core wrap may also be configured to any other suitable configurations known in the art. The core wrap 80 may have a first outer surface 81 facing a wearer-facing surface of an absorbent article and a second outer surface 83 facing a garment-facing surface of an absorbent article. The core wrap 80 may be at least partially overlapped with itself on the second outer surface 83 (see Fig. 10) or the first outer surface 81. The masking material may form a portion of the second outer surface 83. Various components may be at least partially or fully encapsulated within the core wrap 80. The core wrap 80 may comprise a nonwoven material, a tissue material, a portion of the masking material, or other suitable material. Starting on the topsheet side of the absorbent core 24, a first layer 82 may be provided. The first layer 82 may comprise a superabsorbent material 84 and/or a fibrous material, such as cellulosic fibers 86. In some instances, the first layer 82 may only comprise the superabsorbent material 84 or may only comprise the fibrous material 86. If fibrous material 86 86 is provided in the first layer 82, it may help make the wearer-facing side of the absorbent core 26 softer and mask any grittiness caused by the superabsorbent material particles. The first layer 82 may have enough superabsorbent material 84 to enable topsheet dryness, but still may have enough of the fibrous material 86, such as cellulosic fibers, to provide fast bodily exudate acquisition and a soft wearer-facing surface feel.

A hydrophilic nonwoven material or hydrophilic material 88 may be positioned under the first layer 82. A second layer 90 that may comprise a superabsorbent material 84 and/or a fibrous material 86, such as cellulosic fibers, may be positioned under the hydrophilic material 88. In some instances, the second layer 90 may only comprise the superabsorbent material 84 or may only comprise the fibrous material 86. The second layer 90 may have more superabsorbent material than the first layer 82 to provide high storage capacity of bodily exudates while also being thin and, therefore, less bulky. Stated another way, the second layer 90 may have a higher concentration of the superabsorbent material 84 than the first layer 82. The first layer 82 may have a higher concentration of the fibrous material 86 than the second layer 90. The hydrophilic material 88 between the first and second layers 82, 90 may provide capillary continuity between the first and second layers so that bodily exudates may easily transfer from the first layer 82 to the second layer 90. A masking material 92 may be positioned under the second layer 90. The masking material 92 may aid in the softness of the absorbent article from the garment-facing side by masking the potentially gritty feel of the superabsorbent material in the first and/or second layers 82, 90. The masking material 92 may comprise a nonwoven material, a woven material, a fibrous material, such as cellulosic fibers, a film, a foam, a tissue, a high-loft tissue, a fibrous superabsorbent web, and/or any other suitable material. In some configurations, the masking material 92 may form a portion of an outer perimeter of the absorbent core.

Any or all of the first and second layers 82, 90, the hydrophilic material 88, the core wrap 80, and the masking material 92 may have the shape 94 illustrated in Fig. 12 or a similar shape. This shape is considered an extended hourglass shape and allows the crotch region of the absorbent articles to be quite narrow. In the premature infant context, a wide crotch region may not be desired as it may push the infant's legs apart and potentially create issues with hips. Instead of having the extended hourglass shape, notches or recesses may be created in the various components to create a narrow crotch region.

The first layer 82 may have a first width (direction along central lateral axis 16) proximate to its longitudinal end and a second width at its longitudinal midpoint. The first width may be greater than the second width. Likewise, the second layer may have a first width proximate to its longitudinal end and a second width at its longitudinal midpoint. The first width may be greater than the second width. This feature may also apply to the core wrap, the hydrophilic material and/or the masking material. In such instances, any or all of the layers or materials may not be rectangular and may instead be shaped (i.e., not rectangular or square).

The hydrophilic material may be wider than or not as wide (direction along the central lateral axis 16) as the first layer 82, the second layer 90, and/or the masking material 92.

The first layer 82 may comprise the superabsorbent material in the range of about 5% to about 60%, about 5% to about 55%, about 15% to about 55%, or about 20% to about 50%, by weight of the first layer, specifically reciting all 0.5% increments within the specified ranges and all ranges formed therein or thereby. The second layer 82 may comprise the superabsorbent material in the range of about 30% to about 100%, about 40% to about 90%, about 40% to about 80%, about 40% to about 70%, or about 40% to about 60%, by weight of the second layer, specifically reciting all 0.5% increments within the specified ranges and all ranges formed therein or thereby.

The first layer 82 may comprise the fibrous material, such as cellulosic fibers, in the range of about 30% to about 100%, about 40% to about 95%, about 50% to about 90%, or about 50% to about 80%, by weight of the first layer, specifically reciting all 0.5% increments within the specified ranges and all ranges formed therein or thereby. The second layer 82 may comprise the fibrous material, such as cellulosic fibers, in the range of about 30% to about 80%, about 30% to about 70%, about 35% to about 65%, or about 40% to about 60%, by weight of the second layer, specifically reciting all 0.5% increments within the specified ranges and all ranges formed therein or thereby.

The masking material 92 may have a higher basis weight than a basis weight of each of the hydrophilic material 88 and the core wrap 80. The masking material may be formed of one or more layers. A single layer may be folded over itself or multiple individual layers may be provided. The masking material may have a basis weight in the range of about 8 gsm to about 100 gsm, about 10 gsm to about 90 gsm, about 10 gsm to about 60gsm, about 10 gsm to about 40gsm, about 10 gsm to about 30gsm, about 13 gsm to about 25 gsm, or about 13 gsm to about 20 gsm, specifically reciting all 0.5gsm increments within the specified ranges and all ranges formed therein or thereby.

The masking materials of the present disclosure may have a thickness in the range of about 0.25mm to about 20mm, about 0.25mm to about 15mm, about 0.5mm to about 12mm, about 0.5mm to about 11mm, about 1mm to about 10mm, about 1mm to about 8mm, about 2mm to about 6mm, about 3mm to about 5mm, or about 4mm, specifically reciting all 0.01mm increments within the specified ranges and all ranges formed therein or thereby. Thickness is measured according to the Thickness Test herein.

The masking materials of the present disclosure may have a stiffness in the range of about 50mN to about 3,500mN, 100mN to about 3,500mN, about 100mN to about 3,200mN, about 100mN to about 2,500mN, about 100mN to about 2,000mN, about 100mN to about 1,500mN, or about 100mN to about 1,000mN, about 100mN to about 750mN, about 200mN to about 600mN, about 300mN to about 500mN, or about 400mN, specifically reciting all 1mN increments within the specified ranges and all ranges formed therein or thereby. Stiffness is measured according to the Stiffness Test herein.

In an example, a masking material may have a thickness in the range of about 1mm to about 8mm and a stiffness of about 100mN to about 1,000mN. Typically, the thickness should be large enough to mask any gritty feeling of superabsorbent polymers in the absorbent core, while having a low enough of a stiffness to allow the absorbent article to remain flexible and conform to a wearer.

In an example, the masking material may have a basis weight in the range of about 5 gsm to about 75gsm, about 10 gsm to about 65 gsm, about 10 gsm to about 60 gsm, about 10 gsm to about 50 gsm, about 10 gsm to about 45 gsm, about 10 gsm to about 40 gsm, about 15 gsm to about 35 gsm, about 15 gsm to about 30 gsm, about 15 gsm to about 25 gsm, or about 20 gsm, specifically reciting all 0.1 gsm increments within the specified ranges and all ranges formed therein or thereby. Basis weight is measured according to the Basis Weight Test herein.

Referring to Figs. 10 and 11, adhesives may or may not be positioned intermediate the various components. Adhesives may be positioned only between some components and not other components.

At least two of, or at least three of, the core wrap 80 (one or two layers), the hydrophilic material, the masking material 92, and/or other nonwoven materials in the absorbent core 24 may have the same or different properties. These properties may be surface energy, pore size, basis weight, caliper, and/or other nonwoven properties. This may apply in a single material core wrap context (e.g., Fig. 10) or a dual material core wrap context (e.g., Fig. 11).

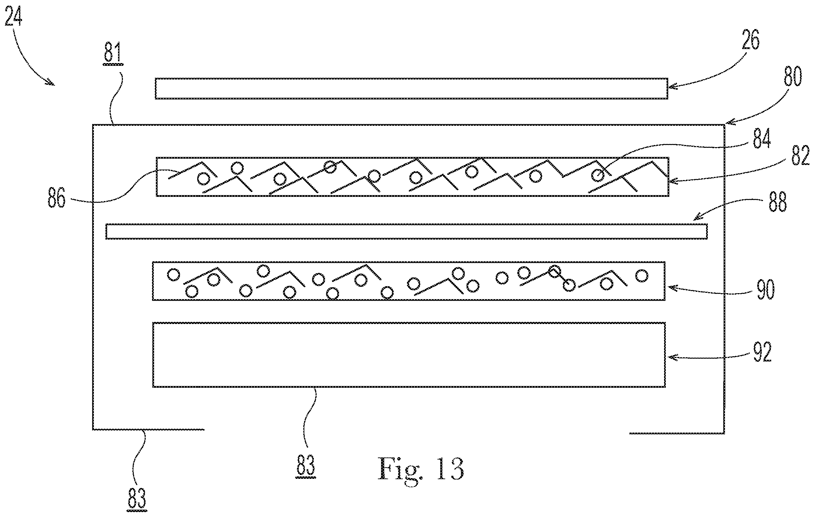

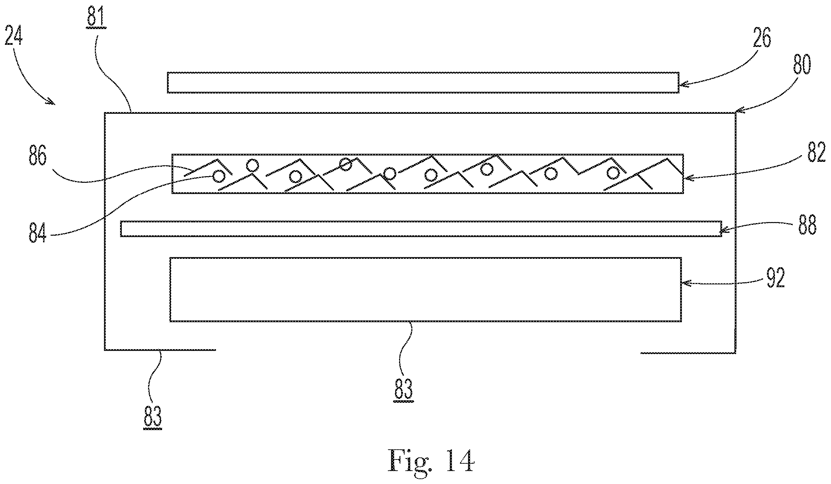

Fig. 13 is an example cross-sectional schematic illustration of an absorbent core 24 and optional acquisition material 26 of the absorbent articles herein, taken about a central lateral axis 16 of a chassis 12. Fig. 14 is another example cross-sectional schematic illustration of an absorbent core 24 and an optional acquisition material 26 of the absorbent articles disclosed herein, taken about a central lateral axis 16 of a chassis 12. In Figs. 13 and 14, the masking material 92 may form a portion of the second outer surface 83 or outer perimeter of the core wrap 80. Stated another way, the core wrap 80 may only partially encapsulate the various components with the masking material 92 completing the encapsulation. Thus, portions of the masking material 92 may be considered a portion of the core wrap. In Figs. 13 and 14, the various components having the same references numbers as Figs. 10-12 may be the same as described above with respect to Figs. 10-12. In Fig. 14, the second layer 90 may not be present.

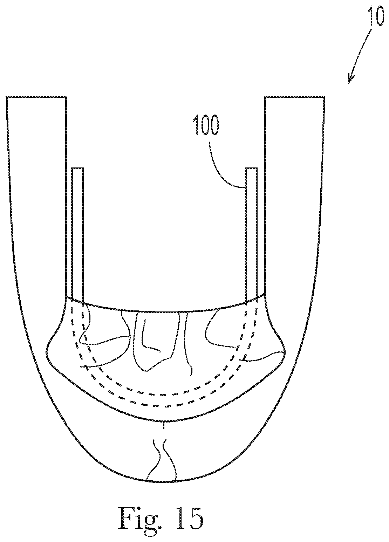

Referring to Figs. 15-20, one or more inserts 100 may be provided for use with an absorbent article 110 (or absorbent article 10 discussed herein). The one or more inserts may be packaged together with one or more absorbent articles or packaged and sold separately. One or more inserts and one or more absorbent articles may be in a kit. The one or more inserts may be positioned within the absorbent article after the absorbent article is donned on the wearer or before the absorbent article is donned on the wearer. The inserts essentially provide an additional absorbent containment element that may be used prior to soiling of the absorbent article or after soiling of the absorbent article. The inserts may be advantageous in that the baby or wearer may not need to be fully changed and, thereby, moved, lifted, or handled less, compared to if only the absorbent article was used. Multiple inserts may be used at one time, so that once soiled the most wearer-facing insert may be removed. In some instances, the inserts may be fastening or joined to the wearer-facing surface of the absorbent article or to a wearer-facing surface of another insert using any suitable joining techniques. In other instances, the inserts may merely be placed on the wearer-facing surface of the absorbent article or the wearer-facing surface without some separate attachment or joining mechanism. In these instances, the inserts may remain in place by friction or merely by weight of the baby. In some instances, at least portion of the inserts may have low coefficients of friction to allow them to slide over a portion of a wearer-facing surface of the absorbent article, when being inserted into the absorbent article while it is on a wearer. The inserts are helpful as full absorbent article changes may be reduced, thereby leading to less stress on (i.e., less movement/lifting of) a baby or premature baby.

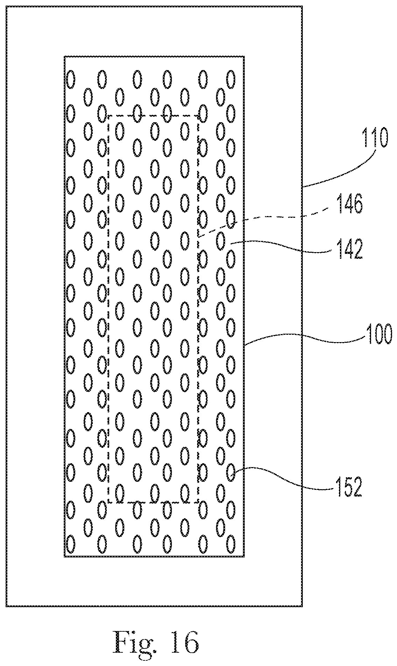

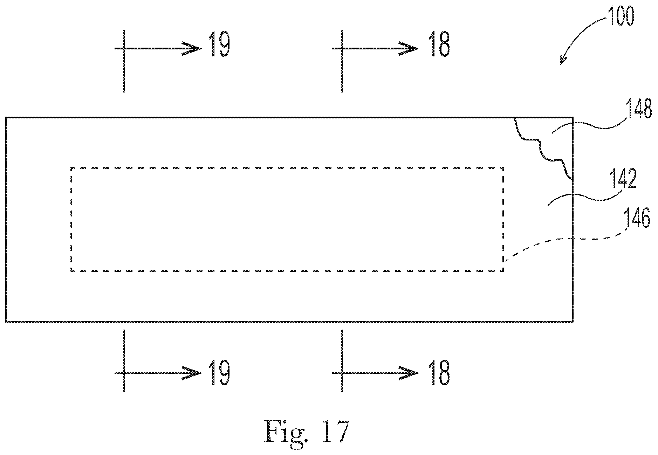

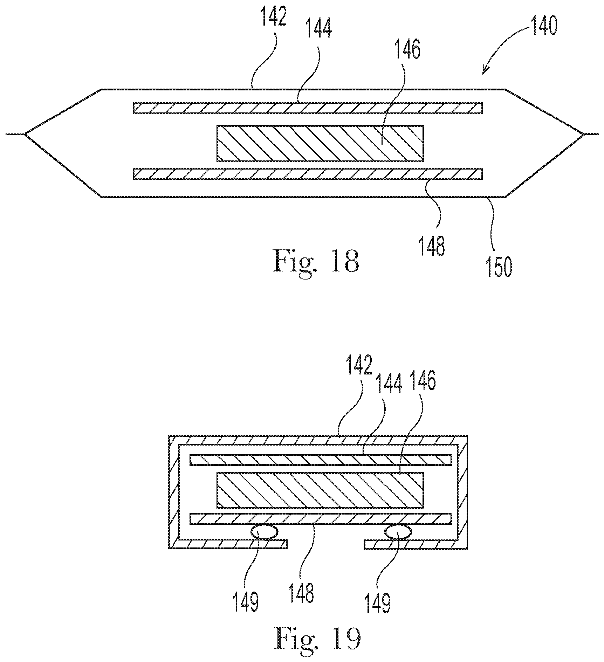

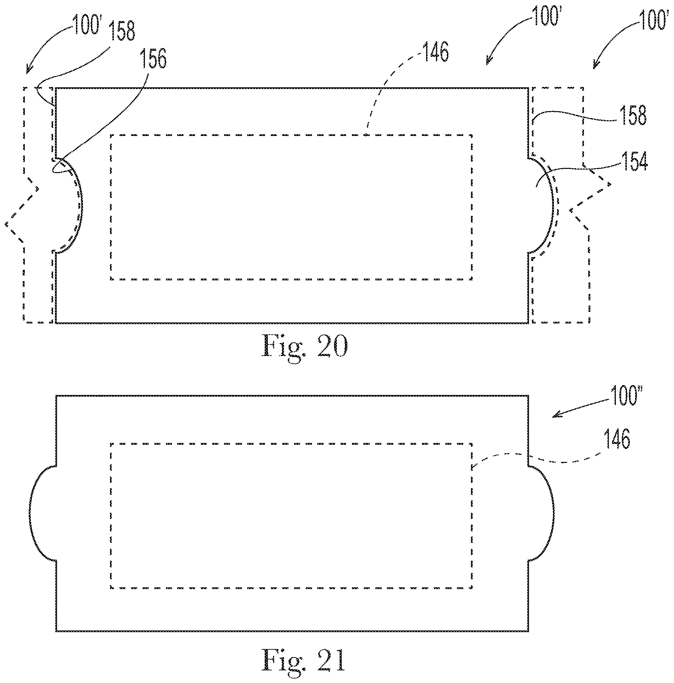

Fig. 16 is a plan view of an insert 100 positioned on a wearer-facing surface of an example absorbent article 110, although the absorbent article may have the outer perimeter and/or other features of the absorbent article 10 discussed herein. Fig. 17 is a plan view of the insert 100. Fig. 18 is an example cross-sectional illustration of the insert 100 of Fig. 17, taken about line 18-18. Fig. 19 is another example cross-sectional illustration of the insert 100 of Fig. 17, taken about line 19-19. Fig. 20 is a plan view of another insert 100'. Fig. 21 is a plan view of another insert 100".

The inserts 100 may comprise a topsheet 142, an optional acquisition material and/or distribution material 144, an absorbent core 146, a backsheet 148, and optionally an outer cover nonwoven material 150. The topsheet 142 may be formed of one or more nonwoven materials, one or more films, or a laminate formed of one or more nonwoven materials and one or more films. The topsheet 142 may comprise a liquid permeable material or apertures 152 defined in the topsheet 142. The topsheet 142 may also comprise three-dimensional features, embossments, bond patterns, graphics, lotions, and/or surfactants, for example. The acquisition material and/or distribution material 144 may be formed of nonwoven materials, foams, or other suitable materials. Optionally, a layer of cross-linked cellulosic fibers may also be present intermediate the topsheet 142 and the acquisition material and/or distribution material 144. The absorbent core 146 may comprise an absorbent material. The absorbent material may comprise a mixture of airfelt and superabsorbent polymers, mostly or only airfelt, or mostly or only superabsorbent polymers. The absorbent core 146 may have areas that are free of the absorbent material (e.g., channels that are free of the absorbent material) or may have areas where the absorbent material is densified (e.g., channels that are formed by densified areas in the absorbent material). The backsheet 148 may be liquid impermeable, and may or may not be air permeable (or "breathable"). In some instances, the backsheet 148 may not be provided and bodily exudates that are not absorbed by the absorbent core 146 may pass through the insert 100 and into the absorbent article 100. The outer cover nonwoven material 150 may or may not be provided and may be formed of a nonwoven material, for example.

Referring to Fig. 19, the insert 100 may have the cross-section illustrated when taken about line 19-19 of Fig. 17. In such an instance, the topsheet 142 may form a C-wrap around end regions of the backsheet 148 and be joined to the backsheet 148 using adhesive bonds 149, or other types of joining. In such an instance, a portion of the backsheet 148 may face the topsheet of an absorbent article in use. Stated another way, a portion of the backsheet 148 may form a garment-facing surface of the insert. An optional acquisition material and/or distribution material 144 may be provided. The insert of Fig. 19 may, in some instances, have an outer cover nonwoven material as well.

Referring to Fig. 20, an insert 100' is illustrated. This example insert 100' has a grasp tab 154 on one end and a cut out 156 on the other end. The grasp tab 154 may be used by a caregiver to easily grasp and remove the insert 100' from an absorbent article after being soiled. The grasp tab 154, in an example, may be formed only of a backsheet material, or other liquid impermeable material, so that bodily exudates cannot wick to the grasp tab 154. In other instances, the grasp tab 154 may be formed of a portion of the topsheet 142, a portion of the backsheet 148, and/or a portion of the outer cover nonwoven material 150. By providing the grasp tab 154 on one end and the cut out 156 on the other end, a material savings may be achieved when the inserts are manufactured in a strip of a plurality of the inserts 100'. Any of the inserts described herein may be manufactured in a strip of a plurality of the inserts and may be packaged as a roll of inserts, for example. Lines of weakness 158 may be formed intermediate the various inserts. In such an instance, a roll of a plurality of inserts may be provided to a caregiver or nurse and the caregiver or nurse can then tear off any suitable number of inserts for use on a particular wearer. The inserts may also be packaged in stacks, for example, or in other forms.

Referring to Fig. 21, the insert 100" is illustrated. The example insert 100" may have a grasp tab 154 on both ends to allow for easier caregiver or nurse placement and removal of the inserts 100".

In some instances, the various inserts may comprise stiffening members or stiffened portions (e.g., foams, densified regions) to aid in application of the inserts into a donned absorbent article.

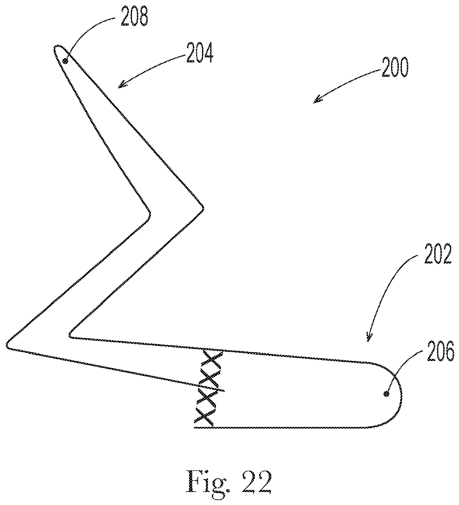

Fig. 22 is an example side view of a leg cuff system 200 with an outer cuff 202 and an inner cuff 204 that may be used in the absorbent articles described herein. The outer cuff 202 may comprise one or more elastic strands 206 and the inner cuff 204 may comprise one or more elastic strands 208. The leg cuff system 200 may be formed of a single piece of nonwoven material or multiple pieces of nonwoven material. If formed of a single piece of nonwoven material, ends or portions of the nonwoven material may be joined together by hem bonds (represented by X's in Fig. 22). It is noted that the overlap at the ends of the nonwoven material proximate to the hem bonds may be reversed. The inner cuff 204 and the outer cuff 202 may each form finished edges owing to the fold over of the nonwoven material. Finished cuff edges are desired for softness and premium visual appearance.

Although the nonwoven material of the leg cuffs herein may be formed of a single piece, it may comprise multiple layers. The layers may comprise one or more spunbond high loft nonwoven layers, one or more melt blown nonwoven layers, and/or one or more spunbond nonwoven layers. Spunbond high loft nonwoven layers typically comprise bicomponent fibers with two different polymers having different melting temperatures. When the polymers cool, one of the polymers cools faster than the other polymer resulting in the fibers curling or "crimping". Spunbond high loft nonwoven layers are also known as crimped nonwoven layers to those of skill in the art.

A nonwoven material for the leg cuffs herein may comprise, in order from the wearer facing surface outward, a first spunbond high loft nonwoven layer, a first meltblown nonwoven layer, a second meltblown nonwoven layer, and a second spunbond high loft nonwoven layer. A cuff, such as the inner or outer cuff 202, 204, for example, may have two of these nonwoven materials folded over themselves. Another nonwoven material for the leg cuffs herein may comprise, in order from the wearer facing surface outward, a spunbond high loft nonwoven layer, a first meltblown nonwoven layer, a second meltblown nonwoven layer, and a spunbond nonwoven layer. A cuff, such as the inner or outer cuff 202, 204, for example, may have two of these nonwoven materials folded over themselves. Yet another nonwoven material for the leg cuffs herein may comprise, in order from the wearer facing surface outward, a first spunbond high loft nonwoven layer, a first meltblown nonwoven layer, a second meltblown nonwoven layer, a second spunbond high loft nonwoven layer, and a spunbond nonwoven layer. A cuff, such as the inner or outer cuff 202, 204, for example, may have two of these nonwoven materials folded over themselves. Still another nonwoven material for the leg cuffs herein may comprise, in order from the wearer facing surface outward, a first spunbond nonwoven layer, a first meltblown nonwoven layer, a second meltblown nonwoven layer, a second spunbond nonwoven layer, and optionally another spunbond nonwoven layer. A cuff, such as the inner or outer cuff 202, 204, for example, may have two of these nonwoven materials folded over themselves.

The spunbond high loft nonwoven layers described above may have a basis weight in the range of about 5 gsm to about 20 gsm, about 7 gsm to about 15 gsm, about 8 gsm to about 13 gsm, about 8 gsm to about 11 gsm, about 9 gsm to about 10.5 gsm, or about 9.9 gsm, specifically reciting all 0.1 gsm increments within the specified ranges and all ranges formed therein or thereby. The meltblown nonwoven layers described above may have a basis weight in the range of about 0.5 gsm to about 5 gsm, about 0.5 gsm to about 3 gsm, about 0.5 gsm to about 2 gsm, about 1 gsm to about 1.5 gsm, or about 1.1 gsm, specifically reciting all 0.1 gsm increments within the specified ranges and all ranges formed therein or thereby. The spunbond nonwoven layers described above may have a basis weight in the range of about 3 gsm to about 20 gsm, about 3 gsm to about 15 gsm, about 4 gsm to about 12 gsm, or about 5 gsm to about 10 gsm, specifically reciting all 0.1 gsm increments within the specified ranges and all ranges formed therein or thereby. Basis Weight is measured according to the Basis Weight Test herein.

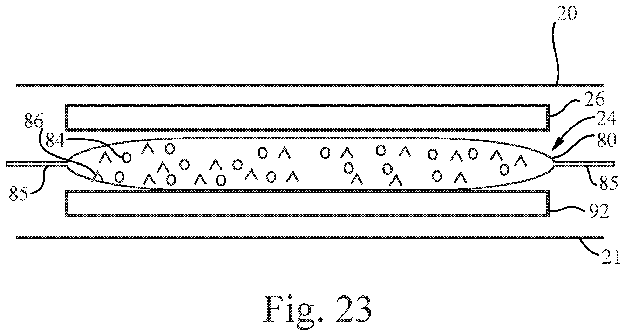

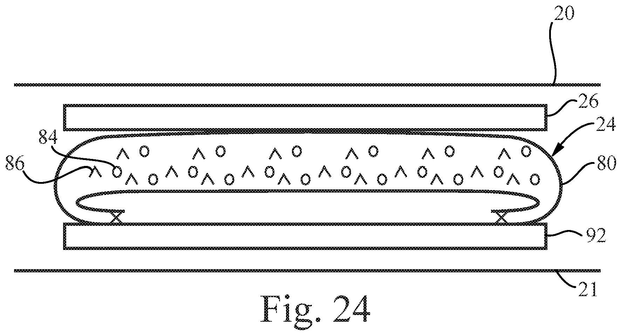

Figs. 23 and 24 are example cross-sectional illustrations (without an outer cover nonwoven material and the fastening members 14 illustrated) of a chassis configuration. Fig. 23 is taken about the central longitudinal axis 18 of Fig. 3 and Fig. 24 is taken about the central lateral axis 16 of Fig. 3. The illustrated components include a topsheet 20, a backsheet 21, an acquisition material 26, an absorbent core 24, and a masking material 92. The absorbent core 24 may comprise a blend of superabsorbent polymers 84 and a fibrous material, such as cellulosic fibers 86. The absorbent core 24 may be wrapped in a core wrap 80 that may be configured as a C-wrap. Side portions of the materials forming the C-wrap may be joined by a bond or adhesives (represented in Fig. 24 by an "X"). End seals 81 of the absorbent core 24 may be joined together by bond or adhesives. The masking material 92 may be positioned outside of the core wrap 80 and at least partially intermediate the absorbent core 24 and the backsheet 21.

### Test Methods

### Thickness Test

The thickness of a test sample is measured as the distance between a reference platform on which the sample rests and a pressure foot that exerts a specified amount of pressure onto the sample over a specified amount of time. All measurements are performed in a laboratory maintained at 23 °C ± 2 C° and 50% ± 2% relative humidity.

Thickness is measured with a manually-operated micrometer equipped with a pressure foot capable of exerting a steady pressure of 0.5 kPa ± 0.01 kPa onto the test sample. The manually-operated micrometer is a dead-weight type instrument with readings accurate to 0.001 mm. A suitable instrument is Mitutoyo Series 543 ID-C Digimatic, available from VWR International, or equivalent. The pressure foot is a flat ground circular movable face with a diameter of 50 mm. The test sample is supported by a horizontal flat reference platform that is larger than and parallel to the surface of the pressure foot. The system is calibrated and operated per the manufacturer's instructions.

Obtain a test sample by removing it from an absorbent article, if necessary. When excising the test sample from an absorbent article, use care to not impart any contamination or distortion to the test sample layer during the process. Test samples are conditioned at 23 °C ± 2 C° and 50% ± 2% relative humidity for 2 hours prior to testing. To measure thickness, first zero the micrometer against the horizontal flat reference platform. Place the test sample on the platform with the test location centered below the pressure foot. Gently lower the pressure foot with a descent rate of 1.0 mm ± 0.1 mm per second until the full pressure is exerted onto the test sample. Wait 5 seconds and then record the thickness of the test sample to the nearest 0.01 mm. In like fashion, repeat for a total of five replicate test samples. Calculate the arithmetic mean for the Thickness and report to the nearest 0.01 mm.

### Stiffness Test

The stiffness or "hand" of a test sample is determined using NWSP 090.3.R0 (15) with a slot width of 6.0 mm and a 100 gram force load cell. Test samples are ideally 200 x 200 mm in size. Smaller samples can be tested by correcting the results as outlined in the NWSP method (i.e., doubling the results obtained for a 100 x 100 mm sample). All measurements are performed in a laboratory maintained at 23 °C ± 2 C° and 50% ± 2% relative humidity. Test samples are conditioned under these same climatic conditions for 2 hours prior to testing. When excising the test sample from an absorbent article, use care to not impart any contamination or distortion to the test sample layer during the process. A total of 5 replicate samples are tested and the arithmetic mean for Stiffness is reported to the nearest 0.1 mN.

### Basis Weight Test

Basis weight of the materials described herein may be determined by several available techniques, but a simple representative technique involves taking an absorbent article or other consumer product, removing any elastic which may be present and stretching the absorbent article or other consumer product to its full length. A punch die having an area of 45.6 cm² is then used to cut a piece of the material being measured from the approximate center of the absorbent article or other consumer product in a location which avoids to the greatest extent possible any adhesive which may be used to fasten the material to any other layers which may be present and removing the material from other layers (using cryogenic spray, such as Cyto-Freeze, Control Company, Houston, Texas, if needed). The sample is then weighed and dividing by the area of the punch die yields the basis weight of the material. Results are reported as a mean of 5 samples to the nearest 0.1 gram per square meter (gsm).

## Claims

1. An absorbent article comprising:
a chassis (12) and one or more fully removable fastening members (14); the chassis (12) comprising:
a topsheet (20);
a backsheet (21);
an absorbent core (24) positioned at least partially intermediate the topsheet (20) and the backsheet (21);
a central lateral axis (16);
a central longitudinal axis (18);
a pair of leg cuffs (70);
a first side edge (28) comprising a first curvilinear portion (36), wherein the first side edge (28) is positioned on a first side of the central longitudinal axis (18);
a second side edge (30) comprising a second curvilinear portion (38), wherein the second side edge (30) is positioned on the second side of the central longitudinal axis (18);
a first waist edge (32) comprising a first curvilinear portion (40), wherein the first waist edge (32) is positioned on a first side of the central lateral axis (16);
a second waist edge (34) comprising a second curvilinear portion (42), wherein the second waist edge (34) is positioned on a second side of the central lateral axis (16); and
wherein the chassis (12) has an outer perimeter, and wherein 50% to 95% of the outer perimeter is curvilinear, and wherein only 5% to only 50% of the outer perimeter comprises linear portions;
wherein the first curvilinear portion (40) of the first waist edge (32) comprises a first concave portion and a first convex portion both with respect to the central lateral axis (16), and wherein the second curvilinear portion (42) of the second waist edge (34) comprises a second concave portion and a second convex portion with respect to the central lateral axis (16); and
**characterized in that** the first waist edge (32) is substantially symmetrical to the second waist edge (34) about the central lateral axis (16), and wherein the first side edge (28) is substantially symmetrical to the second side edge (30) about the central longitudinal axis (18).

2. The absorbent article according to claim 1, comprising:
a first protrusion attached proximate to the first side edge (28);
a second protrusion attached proximate to the second side edge (30);
a third protrusion attached proximate to the first side edge (28); and
a fourth protrusion attached proximate to the second side edge (30).

3. The absorbent article according to Claim 1, wherein the chassis (12) is a uni-body chassis, the chassis (12) comprising:
a first protrusion formed in the first side edge (28);
a second protrusion formed in the second side edge (30);
a third protrusion formed in the first side edge (28); and
a fourth protrusion formed in the second side edge (30);
wherein the first, second, third, and fourth protrusions are not separate elements from the uni-body chassis.

4. The absorbent article according to Claim 3, wherein at least two of the first, second, third, and fourth protrusions comprise an additional curvilinear portion.

5. The absorbent article according to any one of the preceding claims, comprising a first removable fastening member (14) comprising:
a first surface (52);
a second surface (54);
a first fastener (60) on the first surface (52); and
a second fastener (62) on the first surface (52);
wherein the first fastener (60) is configured to be removably engaged with a portion of the chassis on a first side of the central lateral axis (16), and wherein the second fastener (62) is configured to be removably engaged with a portion of chassis on a second side of the central lateral axis (16).

6. The absorbent article according to Claim 5, comprising a second removable fastening member comprising:
a third surface;
a fourth surface;
a third fastener on the third surface; and
a fourth fastener on the third surface;
wherein the third fastener is configured to be removably engaged with a portion of the chassis on a first side of the central lateral axis (16), and wherein the fourth fastener is configured to be removably engaged with a portion of the chassis on a second side of the central lateral axis (16).

7. The absorbent article according to any one of the preceding claims, wherein all portions of the outer perimeter are formed by two layers.

8. The absorbent article according to any one of the preceding claims, wherein linear portions of the outer perimeter comprise a fold, and wherein the curvilinear portions of the outer perimeter comprise a cut edge.

9. The absorbent article according to any one of the preceding claims, comprising a masking material (92) positioned at least partially intermediate the absorbent core (24) and the backsheet (21).

10. The absorbent article according to Claim 9, wherein the masking material (92) has a thickness in the range of 1mm to 8mm, according to the Thickness Test.

11. The absorbent article according to Claim 9 or 10, wherein the masking material (92) has a stiffness in the range of 100mN to 1,000mN, according to the Stiffness Test.

12. The absorbent article according to any one of Claims 9-11, wherein the masking material has a basis weight in the range of 20 gsm to 50 gsm, according to the Basis Weight Test.

13. The absorbent article according to any one of the previous claims, comprising a wetness guard (72) joined to a portion of wearer-facing surface of the absorbent article.

## Patentansprüche

1. Absorptionsartikel, umfassend:
eine Grundeinheit (12) und ein oder mehrere vollständig entfernbare Befestigungselemente (14); eine Grundeinheit (12), umfassend:
eine Oberschicht (20);
eine Unterschicht (21);
einen Absorptionskern (24), der wenigstens teilweise zwischen der Oberschicht (20) und der Unterschicht (21) angeordnet ist;
eine Mittelquerachse (16);
eine Mittellängsachse (18);
ein Paar Beinbündchen (70);
einen ersten Seitenrand (28), umfassend einen ersten krummlinigen Abschnitt (36), wobei der erste Seitenrand (28) auf einer ersten Seite der Mittellängsachse (18) angeordnet ist;
einen zweiten Seitenrand (30), umfassend einen zweiten krummlinigen Abschnitt (38), wobei der zweite Seitenrand (30) auf der zweiten Seite der Mittellängsachse (18) angeordnet ist;
einen ersten Taillenrand (32), umfassend einen ersten krummlinigen Abschnitt (40), wobei der erste Taillenrand (32) auf einer ersten Seite der Mittelquerachse (16) angeordnet ist;
einen zweiten Taillenrand (34), umfassend einen zweiten krummlinigen Abschnitt (42), wobei der zweite Taillenrand (34) auf einer zweiten Seite der Mittelquerachse (16) angeordnet ist; und
wobei die Grundeinheit (12) einen äußeren Umfang aufweist und wobei 50 % bis 95 % des äußeren Umfangs krummlinig sind und wobei nur 5 % bis nur 50 % des äußeren Umfangs lineare Abschnitte umfassen;
wobei der erste krummlinige Abschnitt (40) des ersten Taillenrands (32) einen ersten konkaven Abschnitt und einen ersten konvexen Abschnitt umfasst, wobei beide in Bezug auf die Mittelquerachse (16) sind, wobei der zweite krummlinige Abschnitt (42) des zweiten Taillenrands (34) einen zweiten konkaven Abschnitt und einen zweiten konvexen Abschnitt in Bezug auf die Mittelquerachse (16) umfasst; und
**dadurch gekennzeichnet, dass** der erste Taillenrand (32) im Wesentlichen symmetrisch zu dem zweiten Taillenrand (34) um die Mittelquerachse (16) ist, und wobei der erste Seitenrand (28) im Wesentlichen symmetrisch zu dem zweiten Seitenrand (30) um die Mittellängsachse (18) ist.

2. Absorptionsartikel nach Anspruch 1, umfassend:
einen ersten Vorsprung, der nahe dem ersten Seitenrand (28) angebracht ist;
einen zweiten Vorsprung, der nahe dem zweiten Seitenrand (30) angebracht ist;
einen dritten Vorsprung, der nahe dem ersten Seitenrand (28) angebracht ist; und
einen vierten Vorsprung, der nahe dem zweiten Seitenrand (30) angebracht ist.

3. Absorptionsartikel nach Anspruch 1, wobei die Grundeinheit (12) eine Uni-Body-Grundeinheit ist, die Grundeinheit (12) umfassend:
einen ersten Vorsprung, der in dem ersten Seitenrand (28) ausgebildet ist;
einen zweiten Vorsprung, der in dem zweiten Seitenrand (30) ausgebildet ist;
einen dritten Vorsprung, der in dem ersten Seitenrand (28) ausgebildet ist; und
einen vierten Vorsprung, der in dem zweiten Seitenrand (30) ausgebildet ist;
wobei der erste, zweite, dritte und vierte Vorsprung keine separaten Bestandteile von der Uni-Body-Grundeinheit sind.

4. Absorptionsartikel nach Anspruch 3, wobei wenigstens zwei des ersten, zweiten, dritten und vierten Vorsprungs einen zusätzlichen krummlinigen Abschnitt umfassen.

5. Absorptionsartikel nach einem der vorstehenden Ansprüche, umfassend ein erstes entfernbares Befestigungselement (14), umfassend:
eine erste Oberfläche (52);
eine zweite Oberfläche (54);
ein erstes Befestigungsmittel (60) auf der ersten Oberfläche (52); und
ein zweites Befestigungsmittel (62) auf der ersten Oberfläche (52);
wobei das erste Befestigungsmittel (60) konfiguriert ist, um mit einem Abschnitt der Grundeinheit auf einer ersten Seite der Mittelquerachse (16) entfernbar in Eingriff zu stehen, und wobei das zweite Befestigungsmittel (62) konfiguriert ist, um mit einem Abschnitt der Grundeinheit auf einer zweiten Seite der Mittelquerachse (16) entfernbar in Eingriff zu stehen.

6. Absorptionsartikel nach Anspruch 5, umfassend ein zweites entfernbares Befestigungselement, umfassend:
eine dritte Oberfläche;
eine vierte Oberfläche;
ein drittes Befestigungsmittel auf der dritten Oberfläche; und
ein viertes Befestigungsmittel auf der dritten Oberfläche;
wobei das dritte Befestigungsmittel konfiguriert ist, um mit einem Abschnitt der Grundeinheit auf einer ersten Seite der Mittelquerachse (16) entfernbar in Eingriff zu stehen, und wobei das vierte Befestigungsmittel konfiguriert ist, um mit einem Abschnitt der Grundeinheit auf einer zweiten Seite der Mittelquerachse (16) entfernbar in Eingriff zu stehen.

7. Absorptionsartikel nach einem der vorstehenden Ansprüche, wobei alle Abschnitte des äußeren Umfangs durch zwei Schichten ausgebildet sind.

8. Absorptionsartikel nach einem der vorstehenden Ansprüche, wobei lineare Abschnitte des äußeren Umfangs eine Falte umfassen, und wobei die krummlinigen Abschnitte des äußeren Umfangs einen Schnittrand umfassen.

9. Absorptionsartikel nach einem der vorstehenden Ansprüche, umfassend ein Maskierungsmaterial (92), das wenigstens teilweise zwischen dem Absorptionskern (24) und der Unterschicht (21)angeordnet ist.

10. Absorptionsartikel nach Anspruch 9, wobei das Maskierungsmaterial (92) eine Dicke in dem Bereich von 1 mm bis 8 mm, gemäß der Dickenprüfung, aufweist.

11. Absorptionsartikel nach Anspruch 9 oder 10, wobei das Maskierungsmaterial (92) eine Steifigkeit in dem Bereich von 100 mN bis 1.000 mN, gemäß der Steifigkeitsprüfung, aufweist.

12. Absorptionsartikel nach einem der Ansprüche 9 bis 11, wobei das Maskierungsmaterial ein Flächengewicht in dem Bereich von 20 Gramm pro Quadratmeter bis 50 Gramm pro Quadratmeter, gemäß der Flächengewichtsprüfung, aufweist.

13. Absorptionsartikel nach einem der vorstehenden Ansprüche, umfassend einen Feuchtigkeitsschutz (72), der mit einem Abschnitt der trägerseitigen Oberfläche des Absorptionsartikels verbunden ist.

## Revendications

1. Article absorbant comprenant :
un châssis (12) et un ou plusieurs éléments de fixation entièrement amovibles (14) ; le châssis (12) comprenant :
une feuille de dessus (20) ;
une feuille de fond (21) ;
une âme absorbante (24) positionnée au moins partiellement entre la feuille de dessus (20) et la feuille de fond (21) ;
un axe latéral central (16) ;
un axe longitudinal central (18) ;
une paire de revers de jambe (70) ;
un premier bord latéral (28) comprenant une première partie curviligne (36), dans lequel le premier bord latéral (28) est positionné sur un premier côté de l'axe longitudinal central (18) ;
un second bord latéral (30) comprenant une seconde partie curviligne (38), dans lequel le second bord latéral (30) est positionné sur le second côté de l'axe longitudinal central (18) ;
un premier bord de ceinture (32) comprenant une première partie curviligne (40), dans lequel le premier bord de ceinture (32) est positionné sur un premier côté de l'axe latéral central (16) ;
un second bord de ceinture (34) comprenant une seconde partie curviligne (42), dans lequel le second bord de ceinture (34) est positionné sur un second côté de l'axe latéral central (16) ; et
dans lequel le châssis (12) a un périmètre externe, et dans lequel 50 % à 95 % du périmètre externe est curviligne, et dans lequel seulement 5 % à seulement 50 % du périmètre externe comprend des parties linéaires ;
dans lequel la première partie curviligne (40) du premier bord de ceinture (32) comprend une première partie concave et une première partie convexe toutes deux par rapport à l'axe latéral central (16), et dans lequel la seconde partie curviligne (42) du second bord de ceinture (34) comprend une seconde partie concave et une seconde partie convexe par rapport à l'axe latéral central (16) ; et
**caractérisé en ce que** le premier bord de ceinture (32) est sensiblement symétrique au second bord de ceinture (34) autour de l'axe latéral central (16), et dans lequel le premier bord latéral (28) est sensiblement symétrique au second bord latéral (30) autour de l'axe longitudinal central (18).

2. Article absorbant selon la revendication 1, comprenant :
une première saillie fixée à proximité du premier bord latéral (28) ;
une deuxième saillie fixée à proximité du second bord latéral (30) ;
une troisième saillie fixée à proximité du premier bord latéral (28) ; et
une quatrième saillie fixée à proximité du second bord latéral (30).

3. Article absorbant selon la revendication 1, dans lequel le châssis (12) est un châssis unicorps, le châssis (12) comprenant :
une première saillie formée dans le premier bord latéral (28) ;
une deuxième saillie formée dans le second bord latéral (30) ;
une troisième saillie formée dans le premier bord latéral (28) ; et
une quatrième saillie formée dans le second bord latéral (30) ;
dans lequel les première, deuxième, troisième et quatrième saillies ne sont pas des éléments séparés du châssis unicorps.

4. Article absorbant selon la revendication 3, dans lequel au moins deux des première, deuxième, troisième et quatrième saillies comprennent une partie curviligne supplémentaire.

5. Article absorbant selon l'une quelconque des revendications précédentes, comprenant un premier élément de fixation amovible (14) comprenant :
une première surface (52) ;
une deuxième surface (54) ;
un premier élément de fixation (60) sur la première surface (52); et
un deuxième élément de fixation (62) sur la première surface (52);
dans lequel le premier élément de fixation (60) est conçu pour être mis en prise de manière amovible avec une partie du châssis sur un premier côté de l'axe latéral central (16), et dans lequel le deuxième élément de fixation (62) est conçu pour être mis en prise de manière amovible avec une partie du châssis sur un second côté de l'axe latéral central (16).

6. Article absorbant selon la revendication 5, comprenant un deuxième élément de fixation amovible comprenant :
une troisième surface ;
une quatrième surface ;
un troisième élément de fixation sur la troisième surface ; et
un quatrième élément de fixation sur la troisième surface ;
dans lequel le troisième élément de fixation est conçu pour être mis en prise de manière amovible avec une partie du châssis sur un premier côté de l'axe latéral central (16), et dans lequel le quatrième élément de fixation est conçu pour être mis en prise de manière amovible avec une partie du châssis sur un second côté de l'axe latéral central (16).

7. Article absorbant selon l'une quelconque des revendications précédentes, dans lequel toutes les parties du périmètre externe sont formées par deux couches.

8. Article absorbant selon l'une quelconque des revendications précédentes, dans lequel des parties linéaires du périmètre externe comprennent un pli, et dans lequel les parties curvilignes du périmètre externe comprennent un bord de coupe.

9. Article absorbant selon l'une quelconque des revendications précédentes, comprenant un matériau de masquage (92) positionné au moins partiellement entre l'âme absorbante (24) et la feuille de fond (21).

10. Article absorbant selon la revendication 9, dans lequel le matériau de masquage (92) a une épaisseur dans la plage de 1 mm à 8 mm, selon le Test d'épaisseur.

11. Article absorbant selon la revendication 9 ou 10, dans lequel le matériau de masquage (92) a une rigidité dans la plage de 100 mN à 1 000 mN, selon le Test de rigidité.

12. Article absorbant selon l'une quelconque des revendications 9 à 11, dans lequel le matériau de masquage a une masse surfacique dans la plage de 20 g/m² à 50 g/m², selon le Test de masse surfacique.

13. Article absorbant selon l'une quelconque des revendications précédentes, comprenant une protection contre l'humidité (72) reliée à une partie de la surface de l'article absorbant faisant face à l'utilisateur.
